# EUROPEAN PATENT APPLICATION

(11) **EP 3 510 947 A1**
(43) Date of publication of application: **17.07.2019**
(21) Application number: 18151659.2
(22) Date of filing: 15.01.2018
(51) Int. Cl.: A61B 17/16

(54) **REAMER WITH CUTTING HOLES HAVING DIFFERING SHARPNESS**

(71) Applicant: Arthrex Inc, Naples, FL 34108-1945 (US)
(72) Inventor: Michel, Gerlinde, 80999 Munich (DE); Naudin, Stéphane, 82152 Krailling (DE)
(74) Representative: Lohr, Jöstingmeier & Partner

(57) **Abstract**

A cup-shaped reamer has an array of cutting holes which vary in their sharpness such that the cutting holes closest to the reamer shaft axis have the highest sharpness and the most exterior cutting holes have the lowest sharpness.

## Description

### Field of the invention

The present invention relates to the field of surgical tools and, more particularly, to a reamer with specific surface blade configuration to improve drilling capabilities during surgery.

### Description of the related art

During surgical procedures, it is often necessary to approach a specific anatomical location precisely and directly, to avoid unwanted surgical trauma. For example, orthopedic surgery and related trauma care requires bone reaming to allow for implants. These procedures may include hip replacement, knee replacement and shoulder replacement. Important during surgery is to minimize damage and protect surrounding soft tissue.

Bone reamers usually have a good reaming performance in the peripheral area but poor performance in the center. As a result exceeding reaming force is needed to cut through the initial cortical bone (the most exterior hard part of the bone) portion leading to overheating of the bone and of the surrounding area. This causes bone necrosis which is deleterious for good bone ingrowth after surgery. Also during reaming of a bone such as a humeral head with a conventional reamer an uncontrolled overshoot normally occurs once the hard cortical bone has been penetrated.

A system and a method for reaming bones is disclosed in EP 2131746 B2. According to this document conventional reamers can cut cortical bone initially but can quickly dull after a single use, or at best a few uses. Once the reamer has dull cutting areas, it reduces the efficiency of bone cutting and in addition generates sufficient friction/heat to damage or kill the surrounding bone. The problem is here solved by employing a hollow reamer for medical applications having a disposable hollow cutter assembly which can be attached to a reusable shaft portion, whereby after one use of the reamer, a new hollow cutter assembly is utilized and the used hollow cutter assembly is discarded. To be noted here is the economical and material wastage occurring after every medical procedure. The reamer is simply disconnected from the shaft and discarded. The reamer as such is not improved but just replaced by a new one.

A further prior art document EP 508710 discloses a reamer with a rotatable body, a drill tip including tip edges, reaming edges joined to the tip edges and extending from said tip edges to form a tunnel. When said body is rotated in bone, flute surfaces are disposed between said tip edges for evacuating the bone. This device solves the problem of more efficient cutting and less heat by the angle of the bore blades. However, the blades are uniformly constant sharp over their entire lengths. As such after a couple of uses these reamers become dull and less efficient at least in the areas having most contact with the bone to the extent that they need to be replaced earlier although the majority of the blades may not have reached their end of use live.

After long scientific research it has been concluded that none of the prior art documents satisfy the need for a reamer which is easy and precise to use without overshooting and deforming the hole when penetrating the cortical bone part, protects the surrounding soft tissue, cuts the bone at a constant speed and is reusable hence more economically viable.

### Summary of the invention

The problem to be solved by the invention is to provide a reamer which solves the problem of overshooting and deforming the hole in the process with aggravating consequences for the implant to be fitted to the bone. A further aspect relates to providing a better and more precise cutting tool of a bone without inflicting further injury to the bone by overheating and overshooting the mark.

The solution of the problem is described in the independent claims. The dependent claims relate to further improvements of the invention.

In an embodiment, a reamer provides variable sharpness. The sharpness decreases with increasing radius from the reamer axis. Therefore, close to the axis is the highest sharpness. Herein a higher sharpness preferably relates to the ability of removing more material and/or removing material with a lower force applied to the reamer.

The result is an embodiment that can reduce poor performance of bone reaming and allows for a constant pressure to be applied to the reamer throughout the entire reaming procedure.

In a preferred embodiment, a reamer device has a reamer cup. The reamer cup is preferably circular or disc like shape with a distal or front part and a proximal or back part. The distal portion of the reamer preferably has a through hole at its center. The proximal part is preferably curved in a bowl like form and is empty with only an elongated reamer shaft protruding through it towards the distal part of the reamer cup. The proximal area of the reamer forms a plane.

The elongated reamer shaft is connected to the back of the reamer and is not protruding through the through hole of the reamer but is extending in the opposite proximal direction. The shaft may be connected at its proximal end to a further tool such as an electric or manual drill. At its distal end the shaft is preferably permanently attached by means of welding to the reamer cup. Preferably the shaft may also be detachably attached to the reamer cup by means of a bayonet system or it may be directly screwed in the back of the reamer cup or it may be fixed with a clamp system or by an array of fixing screws or just one or two screws or any other detachable means. The distal part or front part of the reamer cup preferably has an array of variably placed cutting holes. The cutting holes are preferably placed in an elliptical row. The rows may intertwine each other such that the cutting holes closest placed on a radius around the shaft axis and starting from the central ring run preferably on a radial path between the cutting holes on the opposite side of the elliptical rows. Also preferably the closes cutting holes to the reamer shaft axis have the highest blade sharpness. The further away from the center axis of the shaft the more moderate to low the sharpness of the cutting holes become. The most exterior cutting holes have preferably the lowest sharpness.

A further aspect of the invention relates to a method for drilling a hole into a bone joint. When in use, the reamer is preferably connected to a shaft from the back. The reamer may be placed above the immediate exterior part of a bulbous area of the bone joint to be reamed. With the help of a rotating instrument such as a drill attached to the shaft the reamer rotates around its shaft's axis. The operator preferably applies slight pressure force against the drill. This force is in turn transmitted via the shaft to the bone to be reamed. By applying a continuous and constant pressure, the front cutting area of the reamer cuts off parts of the bone.

Generally, the hardest part of a bone is the cortical part or the exterior part of a bone. In comparison the interior of the bone, the cancellous part, has a spongy consistency which is a lot less dense than the exterior part. Because of the bulbous parabolic form of the bone joint the reamer starts first reaming off parts on the highest point of the bulb of the bone joint. The reamer connects the bone first with its center which is the sharpest part of the reamer. Gradually, more bone of the parabolic bulbous bone joint is shaved or cut off and more area of the bulbous part of the bone joint comes into contact with the blades of the reamer. At some point in time, depending of the shape, thickness and density of the cortical part of the bone to be reamed, the bone is penetrated by the reamer's cutting area. At this point conventional normal reamers with constant sharpness become uncontrollable and a shock or a jerky forward movement is observed. Normally, the operator exerts too much pressure on the drill and overshoots when the hard shell of the bone gives way. This is normally corrected by reducing the exerted pressure force upon the reamer. The reason for this jerky movement is because the same force is applied both to the large parabolic bulbous hard shell bone area as it is applied to the subsequent remaining cancellous or spongy part. Since the reamer blade sharpness is constant and at the same speed the reamer transits suddenly from the hard bone into the soft bone with a far too greater force. In this case the bone is cut instantaneously and the reamer penetrates the cancellous part of the bone before the operator can correct the speed of the drill or of the force applied.

This problem is overcome in the present embodiment by the variable sharpness of the cutting area. The blade is sharper at the center and duller gradually towards the edge of the reamer. Once the hardest exterior cortical part of the bulbous bone is penetrated by the reamer's sharper cutting area, the remaining bone shell continues to be cut at a slower intensity but with a similar speed and force applied by the operator. The sudden change in penetration is alleviated by the blunter exterior cutting area of the reamer blades. As such the operator does not feel a sudden easiness in drilling and has at all time perfect control over the drilling procedure and cannot overshoot or move the reamer in a non-axial direction due to a sudden penetration shock. The reamer may only penetrate the bulbous bone until the proximally offset circular depth limit stop comes into contact with the bone. This is preferably the maximum depth of the reamed hole possible and also the maximum radius of the cut off area.

If the end of the hole is reached, the drilling process may be stopped, for example by switching off a motor rotating the drill. After stopping the drill this may be restarted again, manually by pressing a button. The end of the hole may be detected by visually evaluating the distance between the depth limit stop and the bone.

Preferably the materials used for the shaft and the reamer may be out of titanium, titanium alloy, titanium-aluminum-vanadium, surgical stainless steel, martensitic steel or any stainless steel or alloy thereof.

The instruments, systems and methods may be applied to a variety of fields, for example, sports medicine, implant surgery, trauma, etc.

### Description of Drawings

In the following the invention will be described by way of example, without limitation of the general inventive concept, on examples of embodiment with reference to the drawings.
- Figure 1: shows a lateral view of a cup reamer with a shaft and teeth.
- Figure 2: shows distal view of a cup reamer with teeth placed variably.
- Figure 3: shows a lateral view of a cup reamer in the drilling position of bone.
- Figure 4: shows a lateral view of a cup reamer after drilling into a bone.
- Figure 5: shows a sectional lateral view of a reamer, bone and retro pin in the drilling position.
- Figure 6: shows a sectional lateral view of a reamer, bone and retro pin after drilling into a bone.

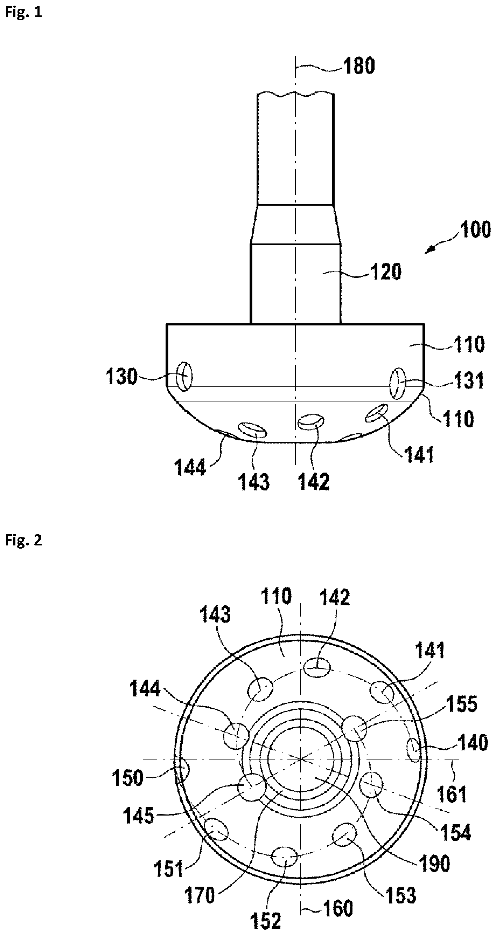

Figure 1 shows a lateral view of an embodiment having a cup reamer 100 with a shaft 120, first cutting holes 140-145 and second cutting holes 150-155. The cup reamer 100 is not flat but has a cup like form. This form allows for the excised bone tissue to be gathered together in the cup and can be easily extracted without leaving any shaved off bone tissue in the work area. The shaft 120 may be permanently fixed or may be detachably attached to the cup 110. The cup has a higher side wall in order to allow for a more precise and deeper reaming.

Figure 2 shows a distal view of a cup reamer 100 with blade cutting holes 140-145, 150-155 placed variably in intertwined positions. Preferably the first bottom blade cutting holes 140-145 are being placed exactly opposite the second bottom blade cutting holes 150-155. Preferably each blade cutting hole follows a different but slightly overlapping radial path. The cup may have a through hole 190 at the center of the cup for allowing other devices or substances being passed down the shaft 120 at the center of the reaming location. In another preferred embodiment the through hole 190 is blocked after a certain distance.

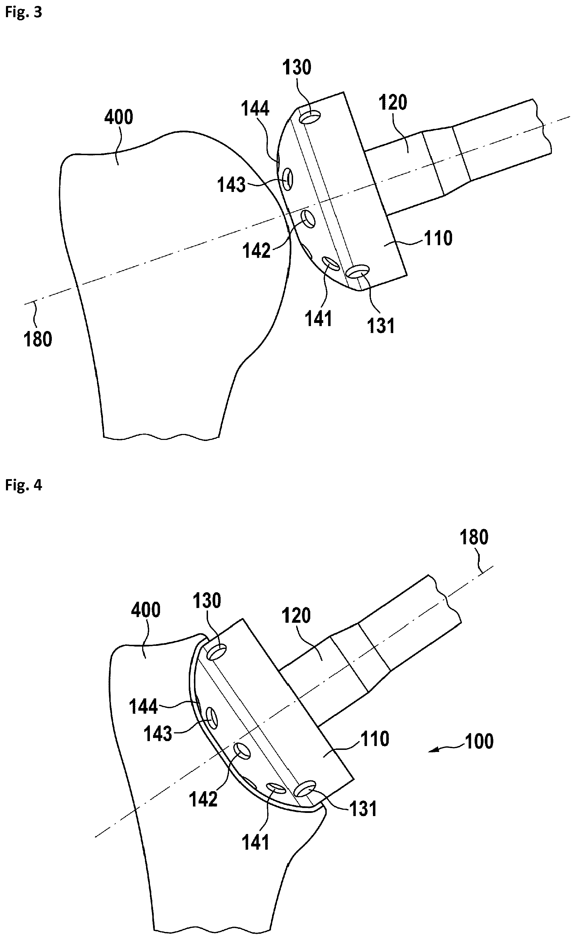

Figure 3 shows a lateral view of a cup reamer 100 in the drilling position of a bone 400. The cup reamer 100 may either be pulled towards the bone with the help of a retro pin 300 or pushed by a shaft 120 as is illustrated in Figure 13. The cup 110 is positioned above the area to be reamed and the reamer 100 may be directed into the bone 400 with the help of a drill bit protruding through the center through hole 190 of the cup 110 or with the help of a radial reamer guide fixed in place on the bone 400, prior to the reaming procedure. The reamer guide may be slightly larger radially than the reamer cup 110 allowing the reamer to be directed towards the bone 400 without sliding of in any direction.

Figure 4 shows a lateral view of a reamer 100 after the drilling procedure into a bone.

In operation the method may have the following steps: placing a reamer 100, 200 above the immediate exterior part of a bulbous part 410 of the bone joint to be reamed and slightly applying a continuous and constant pressure force against the shaft or the retro pin 300 to the bone 400 to be reamed such that the cutting area of the reamer cuts off parts of the cortical area 420 of a bone joint, wherein the reamer 100, 200 connects the bone first with its center most sharp cutting part of the reamer and last with the radially external dull cutting parts.

Furthermore, once the hardest exterior cortical part 420 of the bulbous bone part 410 has been penetrated by the reamer's sharper cutting area, the remaining bone shell continues to be cut at a slower intensity but with a similar speed and force applied by the operator. A sudden change in penetration is alleviated by the blunter or duller exterior cutting area of the reamer blades. The reamer may only penetrate the bulbous bone until the proximally offset circular depth limit stop 210, 211 comes into contact with the bone.

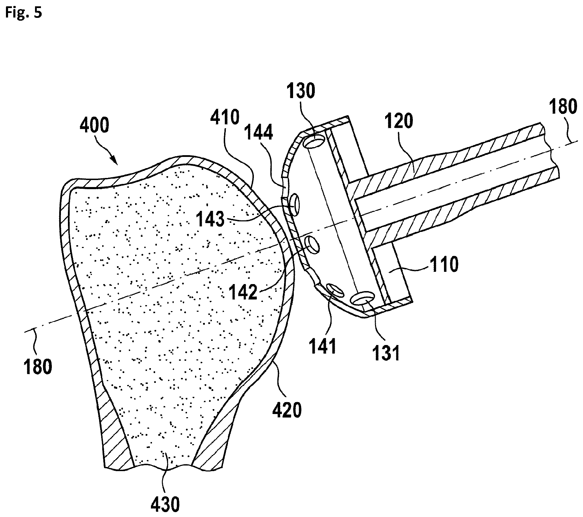

Figure 5 shows a sectional lateral view of a reamer 100 and a bone 400 in the drilling position. Through the sectional view a better understanding can be achieved of the surgical process. For instance, the harder exterior cortical part 420 of the bone can be clearly illustrated. The reamer 100 in this figure was placed above the bulbous part 410 of the bone joint to be reamed.

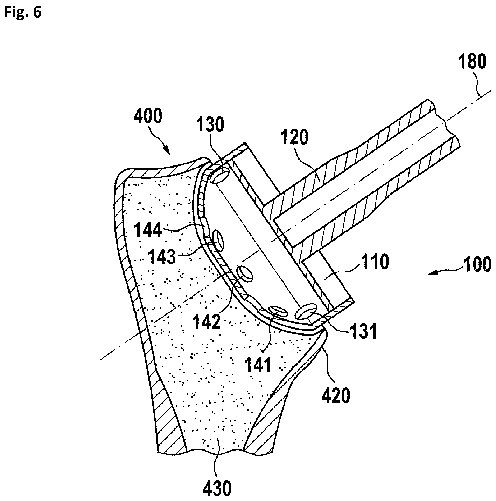

Figure 6 shows a sectional lateral view of a reamer 100 and a bone 400 after drilling into a bone 400 took place. After the surgical step the reamer protruded the exterior cortical part 420 of the bone and entered the spongy or cancellous 430 bone part.

The systems and methods of the present invention allow a single surgeon the ability to operate in a safe and precise environment, to target an area of interest, and to obtain the desired results without any complications such as overshooting or injuring neighboring tissue.

### List of reference numerals

- 100: Cup reamer
- 110: Cup
- 120: Reamer shaft
- 130-131: Side blade holes
- 140-145: First cutting holes
- 150-155: Second cutting holes
- 160: Vertical axis of the cup reamer
- 161: Horizontal axis of the cup reamer
- 170: Centre ring
- 180: Vertical axis of the reamer shaft
- 190: Through hole
- 400: Bone
- 410: Bulbous part
- 420: Cortical bone
- 430: Cancellous bone

## Claims

1. A reamer (100) having a reamer cup (110) and an elongated reamer shaft (120) attached to said reamer cup (110) wherein the reamer cup (110) is circular with a flat distal part and a curved like bowl distal part, wherein said distal part having a through hole (190) at its center
**characterized in that**,
said distal part of the reamer (100) has an array of cutting holes (140-145 and 150-155) placed in elliptically intertwine rows such that the cutting holes (145-144 and 155-154) placed on a radius closest around the shaft axis (180) starting from a central ring (270) when in rotation run radially between the radial path of the holes on the opposite side of each other and said holes (140-145 and 150-155) vary in their sharpness.

2. The reamer (100) according to claim 1,
**characterized in, that**
wherein the cutting holes (145-144 and 155-154) closest to the reamer shaft axis (180) have the highest sharpness and the most exterior cutting holes (140-143 and 150-153) have the lowest sharpness.

3. The reamer (100) according to any one of the previous claims
**characterized in, that**
the materials used for the shaft and the reamer head may be out of titanium, titanium alloy, titanium-aluminum-vanadium, surgical stainless steel, martensitic steel or any stainless steel alloy thereof.
